# EUROPEAN PATENT APPLICATION

(11) **EP 1 929 946 A1**
(43) Date of publication of application: **11.06.2008**
(21) Application number: 05782293.4
(22) Date of filing: 08.09.2005
(51) Int. Cl.: A61B 5/11, A01K 67/00, A61B 5/00

(54) **DEVICE FOR DETECTING HEARTBEAT, RESPIRATION AND BEHAVIOR LEVEL OF SMALL ANIMAL**

(71) Applicant: A.T. Labo, Co., Ltd., Akita-shi, Akita 010-0061 (JP)
(72) Inventor: SATO, Shinichi, Akita-shi Akita 010-0834 (JP)
(74) Representative: Cabinet Plasseraud
(86) International application number: PCT/JP2005/016520
(87) International publication number: WO 2007/029326

(57) **Abstract**

A detection device for detecting heartbeat, respiration and the behavior level of a small animal provided with supporting plate (4), multiple spacers (3) which are located on supporting plate (4) and vibration-transmitting plate (1) which has sensor (2) attached thereto and which is supported on multiple spacers (3).

## Description

### Technical Field

The present invention relates to a detection device for mainly detecting heartbeat, respiration, the behavior level associated with sleep and awakening, and the like of a small animal through a physiological measurement technique.

### Background Art

The present inventors have already proposed a heartbeat and respiration sensor for mainly detecting a heart rate, a respiratory rate and the like of a newborn in Japanese Patent Application No. 2004-319422. The heartbeat and respiration sensor includes: a flexible, thin and sheet-like vibration-transmitting plate; and a piezoelectric transducer attached on a central portion on the back side of the vibration-transmitting plate by using an adhesive or the like. The piezoelectric transducer itself can detect a biomedical signal with high sensitivity, and the vibration-transmitting plate serves to extend the detection range of the biomedical signal by using the piezoelectric transducer. For that reason, a biomedical signal generated by a subject near the heartbeat and respiration sensor as sound, vibration or the like is received by the vibration-transmitting plate, and this causes vibration of the vibration-transmitting plate, which is detected by the piezoelectric transducer, so that the proposed heartbeat and respiration sensor can widely detect the biomedical signal of the subject with high sensitivity without being invasive and without constraining the subject.

However, when the sensor configured as described above is used to detect heartbeat, respiration and the like of a small animal such as a newborn mouse, the following problem arises.

When the sensor described above is used to detect heartbeat, respiration and the like of a small animal, it is conceivable that the sensor is directly laid and installed on the bottom of a cage for housing a small animal. However, the sensor described above is configured in a manner that the vibration-transmitting plate receives a biomedical signal, so vibration of the vibration-transmitting plate caused by receiving the biomedical signal is detected by the piezoelectric transducer, and if the sensor is directly laid on the bottom of a cage for housing a small animal, the vibration cannot be transmitted by the vibration-transmitting plate. Consequently, detection sensitivity of the sensor decreases considerably, and heartbeat and respiration of a small animal may not be detected.

Further, heartbeat sound and respiration sound of a small animal are smaller than those of a human, so it is necessary to use a sensor having a higher detection sensitivity. The sensor configured as described above can be enhanced in its detection sensitivity by making the vibration-transmitting plate thinner. However, if the vibration-transmitting plate is made thinner, the vibration-transmitting plate easily bends, so it may be damaged when handling, for example, when the sensor is laid in or taken out of a cage for a small animal.

Further, during detection of heartbeat, respiration and the like of a small animal, to prevent the body temperature of the small animal from lowering, the sensor preferably has a heater. However, if the heater is mounted on the vibration-transmitting plate, vibration transmission through the vibration-transmitting plate is blocked, so the heater cannot be directly mounted on the vibration-transmitting plate.

### Disclosure of the Invention

An object of the present invention is to provide a detection device for detecting heartbeat, respiration and a behavior level of a small animal which can detect heartbeat, respiration and the like of a small animal with high sensitivity without being invasive and without constraining the small animal, and is easy to handle.

To achieve the object described above, a detection device for detecting heartbeat, respiration and the behavior level of a small animal of the present invention includes: a supporting plate; a plurality of spacers placed on the supporting plate; and a vibration-transmitting plate having a piezoelectric element sensor mounted thereon, supported on the plurality of spacers.

According to the detection device for detecting heartbeat, respiration and a behavior level of a small animal of the present invention, a biomedical signal generated by a small animal placed on the detection device as sound, vibration and the like is received by the vibration-transmitting plate, and vibration of the vibration-transmitting plate caused by receiving the biomedical signal is detected by the piezoelectric element sensor, thereby the biomedical signal of the small animal can be widely detected with high sensitivity without being invasive and without constraining the small animal. Further, the vibration-transmitting plate of the detection device of the present invention is supported on the supporting plate through a plurality of spacers, so vibration transmitted through the vibration-transmitting plate decays less, compared with the case where the vibration-transmitting plate is directly laid on the bottom of a cage, which allows the biomedical signal to be detected with high sensitivity. Further, according to the configuration of the present invention, the vibration-transmitting plate can be prevented from being damaged by grasping the supporting plate during handling, for example, when the detection device is installed on the bottom of a cage and the like for housing a small animal even if the vibration-transmitting plate is made thin to enhance detection sensitivity.

Further, the spacers have a cone-like or pyramid-like shape, and a top of the spacer having the cone-like or pyramid-like shape may support the vibration-transmitting plate. Thus, a contact area of the vibration-transmitting plate with the spacer becomes extremely small, resulting in less decay of the vibration transmitted through the vibration-transmitting plate. Further, the spacer may be formed of an elastic member.

Further, the vibration-transmitting plate may be formed of magnetic material, and a magnet may be provided on a portion of the spacer facing the vibration-transmitting plate, which allows the vibration-transmitting plate to be easily mounted on and removed from the spacer.

Further, a heating means may be provided on the supporting plate. Further, the supporting plate may form a bottom portion of a housing member for housing a small animal.

### Brief Description of the Drawings

Figure 1 is a side view for illustrating one embodiment of a detection device for detecting heartbeat, respiration and the behavior level of a small animal according to the present invention;
Figure 2 is a perspective view of the device shown in Figure 1;
Figure 3 is a view for illustrating an example of installing the detection device in a cage for housing a small animal;
Figure 4 is a side view for illustrating a modification example of the detection device shown in Figure 1 and the like; and
Figure 5 is a perspective view for illustrating another modification example of the detection device shown in Figure 1 and the like.

### Best Mode for Carrying Out the Invention

Next, embodiments of the present invention will be described with reference to the accompanying drawings.

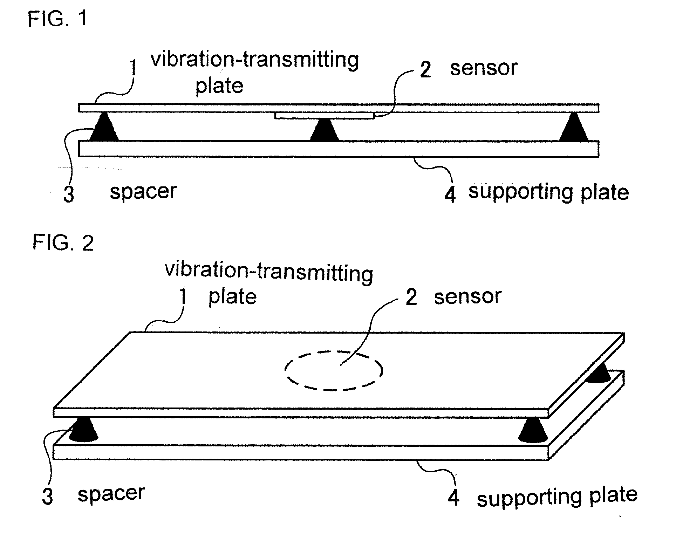

Figure 1 is a side view for illustrating one embodiment of a detection device for detecting heartbeat, respiration and the behavior level of a small animal according to the present invention. Figure 2 is a perspective view of the device shown in Figure 1.

As shown in Figures 1 and 2, the detection device for detecting heartbeat, respiration and the behavior level of a small animal of the present embodiment includes: flexible vibration-transmitting plate 1 to whose central portion on the back side sensor 2 is attached by using an adhesive or the like; and supporting plate 4 for supporting vibration-transmitting plate 1 through a plurality of spacers 3.

Vibration-transmitting plate 1 may be formed of a thin, sheet-like member made of plastic resin or metal. The thickness of vibration-transmitting plate 1 is preferably as thin as possible from the standpoint of transmission good vibrations to sensor 2. On the other hand, a small animal, that is a detection target, is put on vibration-transmitting plate 1, so vibration-transmitting plate 1 has to have a thickness capable of creating a strength sufficient to bear the weight of the small animal. The thickness of vibration-transmitting plate 1 may be appropriately selected depending on the hardness of the raw material for vibration-transmitting plate 1. For sensor 2, for example, a piezoelectric element formed of piezoceramics, or a Poly-Vinyliden-Di-Fluorid (PVDF) film may be used. For sensor 2, in the present embodiment, a circular, piezoelectric transducer having a thickness of about 0.5 mm is used.

Supporting plate 4 has approximately the same size and shape as that of vibration-transmitting plate 1, and may be formed of a member made of plastic resin or metal similarly to vibration-transmitting plate 1. Supporting plate 4 preferably has rigidity and weight to some degree so as to be able to stably support vibration-transmitting plate 1.

Spacer 3 interposed between supporting plate 4 and vibration-transmitting plate 1 is formed of an elastic member, for example, rubber. Five spacers 3 in total, in the present embodiment, are provided at four corners and at a central portion of the detection device, and bonded to supporting plate 4 and vibration-transmitting plate 1 with an adhesive, for example. In a configuration in which spacers 3 are provided only at the four corners of the detection device, vibration-transmitting plate 1 bends so that it becomes depressed when a small animal is in a central region of vibration-transmitting plate 1, and the transmission of vibrations may be blocked because vibration-transmitting plate 1 is brought into contact with supporting plate 4. However, if spacer 3 is also provided at the central region of the detection device, as in the present embodiment, vibration-transmitting plate 1 can be prevented from being depressed. Further, spacer 3 in the present embodiment has a conical shape, and a top thereof supports vibration-transmitting plate 1. Thus, the contact area of vibration-transmitting plate 1 with spacer 3 becomes very small, and the decay of vibrations transmitted through vibration-transmitting plate 1 by spacer 3 is suppressed. The shape of spacer 3, as a matter of course, is not limited to a conical shape, but may be a shape of another cone or pyramid or the like.

According to the detection device of the present embodiment configured as described above, the heartbeat sound and respiration sound of a small animal placed on the detection device, or the sound of footsteps generated when the small animal moves cause vibration-transmitting plate 1 to vibrate, and the vibration is transmitted to sensor 2. Sensor 2 converts deflection generated therein from the vibration into an electrical signal, and outputs it. In addition, vibration-transmitting plate 1 is supported on spacers 3 having an extremely small contact area, so the vibration transmitted through vibration-transmitting plate 1 is only slightly decayed by spacers 3. Thus, the detection device of the present embodiment can satisfactorily detect heartbeat sound, respiration sound, and the like of a small animal even if the small animal is in any place on vibration-transmitting plate 1. The detection device of the present embodiment can detect heartbeat sound, respiration sound, and the like of a small animal without attaching an electrode or the like to the body of the small animal, so the detection device can measure, without being invasive and without constraining the small animal, physiological functions of a small animal, especially such as a newborn mouse to whose body an electrocardiogram electrode, a respiration sensor or the like cannot be attached because the animal is very small.

Further, according to the configuration of the present embodiment, vibration-transmitting plate 1 can be prevented from being damaged by grasping supporting plate 4 during handling, for example, when the detection device is installed on the bottom of a cage and the like for housing a small animal even if vibration-transmitting plate 1 is made thin to enhance detection sensitivity.

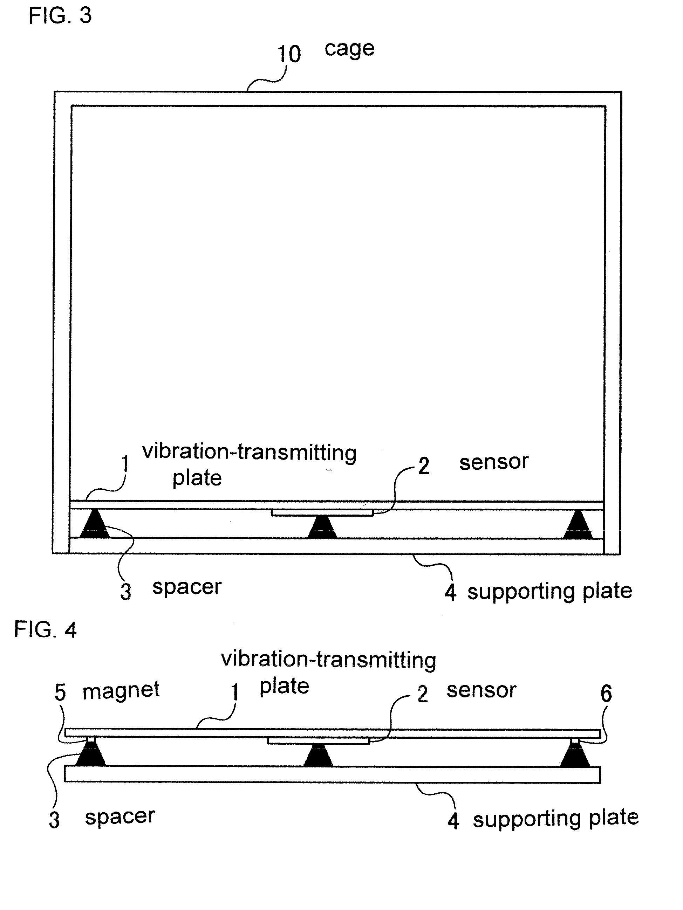

Figure 3 is a view for illustrating an example of installing the detection device in a cage for accommodating a small animal.

In the above description, the case where the detection device is installed on the bottom of a cage or the like has been described, but the detection device and cage 10, as shown in Figure 3, may be integrated with each other so that supporting plate 4 of the detection device forms a bottom portion of the cage or the like. The detection device may be fixed to cage 10, or configured to be removable from cage 10. When the detection device is configured to be removable from cage 10, cleaning, repairing, exchanging, or the like of the detection device is convenient.

Figure 4 is a side view for illustrating a modification example of the detection device shown in Figure 1 and the like. The detection device shown in Figure 4 has magnet 5 on the top of spacer 3 in contact with vibration-transmitting plate 1, and vibration-transmitting plate 1 is formed of magnetic material sot that is is attracted to magnet 5. This vibration-transmitting plate 1 can be easily mounted on and removed from spacer 3, so only vibration-transmitting plate 1 can be removed to facilitate cleaning, repairing, exchanging, or the like.

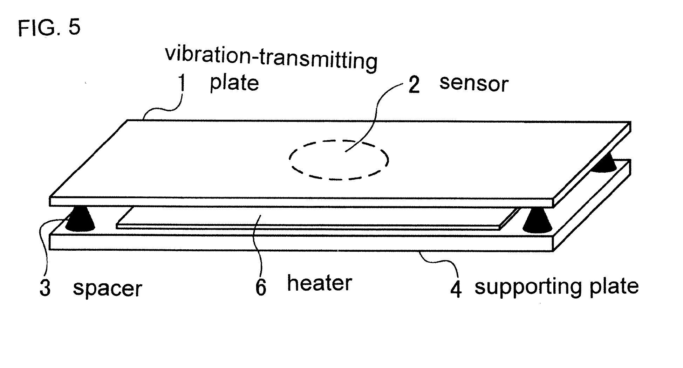

Figure 5 is a perspective view for illustrating another modification example of the detection device shown in Figure 1 and the like. The detection device shown in Figure 5 has sheet-like heater 6 on supporting plate 4. This heater 6 can be used, for example, to prevent the body temperature of a small animal from becoming low at a low air temperature, and to maintain the body temperature of the small animal which becomes low because of receiving anesthesia at a normal physical state. Heater 6 is mounted on supporting plate 4 adjacent to vibration-transmitting plate 1 to be out of contact with vibration-transmitting plate 1, so that the vibration transmitting characteristics of vibration-transmitting plate 1 are not affected at all.

## Claims

1. A detection device for detecting heartbeat, respiration and the behavior level of a small animal, comprising:
a supporting plate,
a plurality of spacers placed on the supporting plate, and
a vibration-transmitting plate having a piezoelectric element sensor attached thereto, supported on the plurality of spacers.

2. The detection device for detecting heartbeat, respiration and the behavior level of a small animal according to claim 1, wherein
the spacer has a cone-like or pyramid-like shape, and
the vibration-transmitting plate is supported on a top of the spacer having the cone-like or pyramid-like shape.

3. The detection device for detecting heartbeat, respiration and the behavior level of a small animal according to claim 1 or 2, wherein
the spacer is formed of an elastic member.

4. The detection device for detecting heartbeat, respiration and the behavior level of a small animal according to claim 1, wherein
the vibration-transmitting plate is formed of magnetic material, and
a magnet is provided on a portion of the spacer facing the vibration-transmitting plate.

5. The detection device for detecting heartbeat, respiration and the behavior level of a small animal according to any one of claims 1 to 4, wherein
a heating means is provided on the supporting plate.

6. The detection device for detecting heartbeat, respiration and the behavior level of a small animal according to any one of claims 1 to 5, wherein
the supporting plate forms a bottom portion of a housing member for housing a small animal.
